# EUROPEAN PATENT SPECIFICATION

(11) **EP 1 531 730 B1**
(45) Date of publication and mention of the grant of the patent: **18.01.2012**
(21) Application number: 03734336.5
(22) Date of filing: 30.05.2003
(51) Int. Cl.: A61B 8/00

(54) **Apparatus for acquiring ultrasound scans of a breast**
Vorrichtung zur Aufnahme von Ultraschallbildern einer Brust
Dispositif d'acquisition d'images à ultrasons

(30) Priority: 31.05.2002 US 160836; 27.11.2002 US 305661; 27.11.2002 US 305936; 09.01.2003 US 439437 P
(43) Date of publication of application: 25.05.2005
(60) Divisional application: 10011891.8
(73) Proprietor: U-Systems, Inc., San Jose, CA 95134 (US); Karssemeijer, Nico, 6573 DZ Beek (NL)
(72) Inventor: WANG, Shih-Ping, Los Altos, CA 94022 (US); RAO, Fangyi, San Jose, CA 95120 (US); CHIN, Donald, Palo Alto, CA 94303 (US); KARSSEMEIJER, Nico, NL-6573DZ Beek (NL); ZHANG, Wei, Union City, CA 94587 (US); YU, Zengpin, Palo Alto, CA 94306 (US)
(74) Representative: Brinck, David John Borchardt
(86) International application number: PCT/US2003/017312
(87) International publication number: WO 2003/101303

(56) References cited:
- DE-A1- 19 753 571
- DE-A1- 19 902 521
- US-A- 5 640 956
- FOSTER F S ET AL: "The ultrasound macroscope: initial studies of breast tissue" ULTRASONIC IMAGING USA, vol. 6, no. 3, July 1984 (1984-07), pages 243-261, XP002414673 ISSN: 0161-7346

## Description

### FIELD

This patent specification relates to medical imaging systems and processes. In particular, this patent specification relates to the acquisition and display of breast ultrasound information in a manner that complements traditional x-ray mammogram-based breast cancer screening methods.

### BACKGROUND

Breast cancer is the most common cancer among women other than skin cancer, and is the second leading cause of cancer death in women after lung cancer. The American Cancer Society currently estimates that there are about 203,500 new invasive cases of breast cancer per year among women in the United States and 39,600 deaths per year from the disease. Prevention and early diagnosis of breast cancer are of foremost importance. Because early breast cancer does not produce symptoms, the American Cancer Society recommends a screening mammogram and a clinical breast examination every year for women over the age of 40.

X-ray mammography is currently the only imaging method for mass screening of breast cancer. In health maintenance organizations (HMO's) and other medical organizations, specialized x-ray mammography clinics designed for high patient throughput are being increasingly used to screen as many women as possible in a time and cost efficient manner. Numerous studies have shown that early detection saves lives and increases treatment options. Recent declines in breast cancer mortality rates (e.g., 39,600 deaths in 2002 versus 41,200 in 2000) have been attributed, in large part, to the regular use of screening x-ray mammography.

Screening x-ray mammography practice in the United States has become largely standardized. For each x-ray mammogram screening of a patient, two standard x-ray mammogram views of each breast are commonly taken: a top (head-to-toe) view ordinarily called the craniocaudal view ("CC"), and a lateral view ordinarily called the mediolateral oblique view ("MLO"). Several efficiencies arise by virtue of this standardization. Importantly, the examinations can be conducted by an x-ray technician instead of a radiologist, with the radiologist later analyzing x-ray mammograms en masse for a large number of patients. An experienced radiologist can achieve a high throughput, e.g., on the order of 2 minutes per patient. This is a key advantage in today's cost-conscious health care environments, because additional radiologist time per patient means additional cost per patient. The efficacy of radiological procedures is today measured by the cost in dollars per quality adjusted life year (QALY), with procedures costing more than $100,000 per QALY being neither encouraged nor prescribed.

Other advantages of x-ray mammogram standardization include: the ability to compare and statistically track large numbers of x-ray mammograms taken from different facilities; the ability to track changes in a single patient over time even if the x-ray mammograms are taken at different facilities; the ability of radiologists to gain recursive expertise in analyzing the standard x-ray mammogram views; and the repeatability of results. The standardization of x-ray mammograms also yields benefits in the public health care area, including the ability for the U.S. government to provide a fixed and predictable per-mammogram reimbursement for Medicare patients. Additionally, health maintenance organizations (HMOs) and other medical insurers are provided with predictable outlays for breast cancer screening of their member patients using x-ray mammography.

A well-known shortcoming of x-ray mammography practice, however, is found in the case of dense-breasted women including patients with high content of fibroglandular tissues in their breasts. Because fibroglandular tissues have higher x-ray absorption than the surrounding fatty tissues, portions of breasts with high fibroglandular tissue content are not well penetrated by x-rays and thus the resulting mammograms would contain little or no information in areas where fibroglandular tissues reside. A study by Lehman et. al., entitled "Effect of Age and Breast Density on Screening Mammograms with False-Positive Findings," on 46,340 patients, published in the December 1999 issue of the American Journal of Reoentgenology (AJR), reports that the proportion of dense breasts (summing those with "heterogeneously dense" and "extremely dense" breasts) account for about 52% of women with age range of 35-39, 47% of age range 40-49, 32% of age range 50-59, 24% of age range 60-69, 23% of age range 70 or older, and 36% for all ages. For the estimated 36% of the female population who have dense breasts, this means that at least a portion of the breast area on the x-ray mammogram cannot be scrutinized for lesions by x-ray mammography alone. As a result, lesions camouflaged by dense breast tissue may go undetected.

Indeed, a study by Kolb et. al. on 18,005 consecutive patients, as reported by Jalali, entitled "Sound Combination: Ultrasound Paired With a Mammography Can Improve Cancer Detection for Dense-Breasted Women," published in the March 1999 issue of ADVANCE for Administrators In Radiology and Radiation Oncology, pages 68-70, states that x-ray mammography alone was able to detect only 70 percent of the cancers (56 of the 80 cancers) in 7,202 patients with dense breasts. Kopans, in a paper entitled "Breast cancer screening with ultrasonography," published in Lancet, Volume 357 (1999), pages 2096-2097, estimates that only 68% of the breast cancers of the screening population would be detected by x-ray mammography alone.

The study by Kolb, *supra,* revealed that by performing ultrasound examination, in the hand-held fashion by a physician and in real-time at a rate of 4 to 20 minutes per patient, on the 7,202 women with dense breasts, an additional 24 percent (19 of the 80 cancers) were detected. X-ray mammography alone detected 70 percent (56 of the 80 cancers), while combining x-ray mammography with ultrasound examination 94 percent of the cancers (75 of the 80 cancers) in dense breasts were detected.

Several other studies showing improved early breast cancer detection using independent ultrasound examination are reported in Jackson, "Controversies in Ultrasound Screening," Society of Breast Imaging 5th Postgraduate Course, San Diego, California, pp. 93-95 (May 2001).

A study by Richter et. al. evaluated an "automated ultrasound system" that generated two automatically reconstructed survey images of the breast based on an acquired set of three-dimensional B-mode scans, and was reported in Richter, K. et. al., "Detection of Malignant and Benign Breast Lesions with an Automated US System: Results in 120 Cases," Radiology 205:823-830 (December 1997). An experimental compression system compressed the breast as in mammography while a motor-driven transducer scanned the breast through an upper compression plate. The two survey images that were constructed from these three-dimensional B-mode scans were (i) a maximum intensity projection (MIP) image mapped from the three-dimensional B-mode scans onto a two-dimensional plane parallel to a lower compression plate, and (ii) a velocity map representing an average acoustic velocity in a direction perpendicular to the lower compression plate. For each patient among a set of patients having known malignant lesions, known benign lesions, or neither, four "blinded" radiologists not involved in the examination of the patients separately examined (i) the two survey images derived from the B-mode scans, with optional access to any of the original B-mode images, and (ii) a corresponding set of conventional x-ray mammograms. It was found that the rate of detection for malignant lesions was 100% (39 of 39 lesions) for combined mammography and ultrasound for all of the radiologists, with the condition that each lesion was identified on at least one of the medical images. The authors stated, "For both benign and malignant lesions, our results show that mammography and ultrasound are complementary modalities; as expected, this does not hold for those lesions that were objectively depicted by means of only one of the two modalities." Richter, *supra* at 830.

Despite strong evidence that use of independent ultrasound examination would improve early breast cancer detection and therefore save lives, substantial resistance against such use currently exists in the medical industry and among policymakers. Jackson, for example, in a paper entitled "The current role of ultrasonography in breast imaging," published in Radiologic Clinics of North America, Volume 33 (1995), pages 1161-1170, states, "The use of ultrasound for breast screening may, however, be harmful to patients." A standard textbook for breast imaging by Heywang-Kobrunner, Dershaw and Schreer, entitled: "Diagnostic Breast Imaging", published in 2001 by Thieme, states on page 88: "Only anecdotal evidence suggests that sonographic screening added to mammography may allow detection of additional carcinomas. However, the existing results suggest that the false positive rate (recommendation for biopsy for lesions that are benign) may be unacceptably high with sonography. The examination is also very operator dependent and time consuming. Feasibility of a quality assurance (technique and reporting), which, however, would be indispensable for any type of ultrasound screening, is not established."

Moreover, the Standards of the American College of Radiology specifically recommend against sonography for breast cancer screening. Heywang-Kobrunner et. al., *supra* at p. 88. The following interrelated factors are often cited against widespread use of ultrasound in breast cancer screening: (i) the false negative (missing) rate of independent ultrasound examination is unknown, (ii) the false positive rate of independent ultrasound examination is known to be very high, leading to an increase in unneeded patient callbacks and biopsies, (iii) lack of image acquisition standardization, leading to variability among different operators and radiologists, (iv) the additional time and equipment required to conduct the ultrasound examination, leading to an increase in cost, and (v) most if not all the breast physicians and radiologists are not trained to read screening ultrasound images, which contain features not found in current breast imaging textbooks or taught in current medical school courses, leading to a potential increase in false negative (missing) rate and in the additional radiologist time required to analyze the ultrasound images, and additional training and clinical experience required for the radiologist to properly analyze the ultrasound images.

Current ad hoc techniques for screening ultrasound examination, as reported by Kolb and others, are indeed not amenable to large-scale integration into the current breast cancer screening environment. For example, in the studies cited *supra* in support of breast screening ultrasound, many of the doctors simply performed the entire screening process themselves, scanning the breast with a hand-held ultrasound probe and viewing the ultrasound display monitor in real-time. Because this usually takes 4 to 20 minutes, such real-time analysis would be cost-prohibitive in today's mass screening environment. The ultrasound viewings are conducted independently on the monitor of the ultrasound machine in real-time without referring to any x-ray mammogram information that may exist for the patient. More importantly, if one pictures the breast as a book, the x-ray mammogram is a picture of the whole book with all the pages of the book superimposed on each another, while the ultrasound images each page independently. The ultrasound image contains many detailed features not observable in an x-ray mammogram, and is very different from a x-ray mammogram in appearance. In addition, the x-ray mammogram is fixed in orientation, either in CC or in MLO views, whereas, as reported by Kolb and others, for example when each breast is scanned in the radial and/or anti-radial fashion around the nipple, each ultrasound image has a different orientation and plane. Thus, even if one wants to view the ultrasound image with an x-ray mammogram, very little can be gained from such practice.

The problem of radiologist skill and training is a particularly important problem to overcome for any breast screening ultrasound scheme to gain acceptance. It has been estimated that only a small portion of today's radiologists would have the ability to effectively use today's ad hoc ultrasound techniques in a mass-screening environment without unacceptable increases in false positives or false negatives.

Vibrational Doppler imaging (VDI) and vibrational resonance techniques, such as those discussed in U.S. Pats. 5,919,139 and 6,068,597 have been proposed for analyzing suspect tumors. As discussed in Lowers, J., "Experimental Modes Abound For Detecting Breast Cancer: Vibrational Resonance Technique Among the Contenders," Women's Health Supplement to Diagnostic Imaging (April 2001) at pp. 15-17, an audio speaker is attached to the ultrasound probe to introduce audio-range vibrational tones (e.g., 69-247 Hz) into the patient during the acquisition of power Doppler ultrasound frames. Different tissue types often vibrate by different amounts responsive to the acoustic signals, and the different vibrations result in different power Doppler readings. Generally speaking, many types of suspect lesions tend to vibrate less than the surrounding breast tissue. It has been found that the absence of vibrations as compared to surrounding tissue can help to clearly differentiate suspect lesions, even those that appear isoechoic (*i.e*., less noticeable) with surrounding tissue on B-scan ultrasound frames. In some clinical practice, the patient is asked to produce her own acoustic vibration by humming at audio frequencies. This practice is called "fremitus." Unless otherwise specified herein, VDI refers generally to color or power Doppler images derived from a breast as it is vibrated at one or more audio frequencies, while vibrational resonance refers to VDI data taken at one or more particular sets of audio frequencies.

In view of the above discussions, it would be desirable to provide an adjunctive ultrasound mammography system that integrates ultrasound mammography into current breast cancer screening methodologies.

It would be further desirable to provide an adjunctive ultrasound mammography system in which the benefits of the many years of professional expertise developed in relation to current x-ray mammography, either the analog or the digital, methods are maintained.

It would be further desirable to provide an adjunctive ultrasound mammography system that takes little or no special familiarization or training from the radiologist in order to effectively view ultrasound information in combination with the x-ray mammogram information.

It would be further desirable to provide an adjunctive ultrasound mammography system in which technicians or assistants may perform the ultrasound scans for later en masse analysis by a physician, the physician's presence not being required during the scanning procedure.

It would be even further desirable to provide an adjunctive ultrasound mammography system in which per-patient image analysis time is not substantially increased as compared to x-ray mammogram analysis alone, or which may even reduce per-patient image analysis time.

It would be still further desirable to provide an adjunctive ultrasound mammography image acquisition system that assures standardization of techniques and minimizes operator variability.

It would be even further desirable to provide an adjunctive ultrasound mammography system that is easy to use, comfortable to the patient, and provides standardized and repeatable ultrasonic scans.

It would be still further desirable to provide an adjunctive ultrasound mammography system that is amenable to two and three dimensional computer-assisted diagnosis (CAD) techniques and to provide several such CAD techniques.

It would be still further desirable to provide an adjunctive ultrasound mammography system that is amenable to combined CAD analysis of ultrasound information with x-ray mammogram information for an enhanced CAD system and to provide several such CAD techniques.

It would be even further desirable to provide an adjunctive ultrasound mammography system for which, upon acquisition of the system, any increase in breast cancer screening costs is offset by savings brought about by an increased early breast cancer detection rate, whereby cost per patient QALY is ultimately reduced. ;;eol;;

It has been found that the use of ultrasound mammography (sonomammogaphy) in conjunction with conventional x-ray mammography can drastically increase the early breast cancer detection rate. Whereas x-ray mammograms only detect a summation of the x-ray opacity of individual slices over the entire breast, ultrasound can separately detect the acoustic impedance of individual slices of breast tissue, and therefore may allow detection of breast lesions where x-ray mammography alone fails.

However, despite strong evidence that use of independent ultrasound examination would improve early breast cancer detection and therefore save lives, substantial resistance against such use currently exists in the medical industry, including the radiologists themselves, and among policymakers. As used herein, the term "radiologist" generically refers to a medical professional that analyzes medical images and makes clinical determinations therefrom, it being understood that such person might be titled differently, or might have differing qualifications, depending on the country or locality of their particular medical environment.

Various schemes have been proposed for processing and presenting breast ultrasound information in conjunction with x-ray mammogram information for use in breast cancer detection environments. In U.S. Pat. 5,938,613, a method and apparatus for performing sonomammography and enhanced x-ray imaging is discussed in which ultrasound equipment is integrated with mammography equipment to generate ultrasonic images of the breast that are in geometric registration with an x-ray mammogram. An x-ray mammogram image of an immobilized breast is acquired and, while the breast is still immobilized, an ultrasound scan is acquired using an automated ultrasound probe translation mechanism. Cross-sectional ultrasonic slices are summed across the entire breast to form a two-dimensional ultrasound image, which is then overlaid onto the digitized x-ray image for viewing by the radiologist. Precise geometric registration between the ultrasound image and the x-ray mammogram is automatically provided because the breast is immobilized between imaging procedures and because the coordinates of the ultrasound probe are known during each scan. The radiologist is permitted to instantiate certain algorithms such as digital subtraction between the registered medical images.

However, the '613 patent is deficient in several respects with respect to the practical, real-world factors associated with the current resistance against the use of ultrasound in mass breast cancer screening environments. For example, the large base of currently installed x-ray imaging systems would require substantial retooling to accommodate the mechanical apparatus of the '613 patent that keeps the breast immobilized between imaging procedures and that performs the automated ultrasound scans. As another example, by displaying a summation ultrasound image of all breast slices together, the '613 method deprives the radiologist of the ability to view individual planes inside the breast. More generally, the computer-registered, static overlay of the summation ultrasound image onto the x-ray image affords only a limited amount of ultrasonic information to the radiologist as compared to the actual amount of ultrasonic data actually acquired, and affords only limited perception by the radiologist of structures within the breast.

In U.S. Pat. 5,662,109, a method and system for multi-dimensional imaging and analysis for early detection of diseased tissue is discussed. Ultrasound scans of a breast are processed into multiple layers of two-dimensional images, thus yielding a three-dimensional data set. This data set and a two-dimensional x-ray mammogram are input to an enhancer that performs one or more "data fusion" algorithms to generate a three-dimensional representation of the breast for viewing. The enhancer includes a registration module that expands and/or reduces dimensions of the data to register and align the ultrasound and mammographic images.

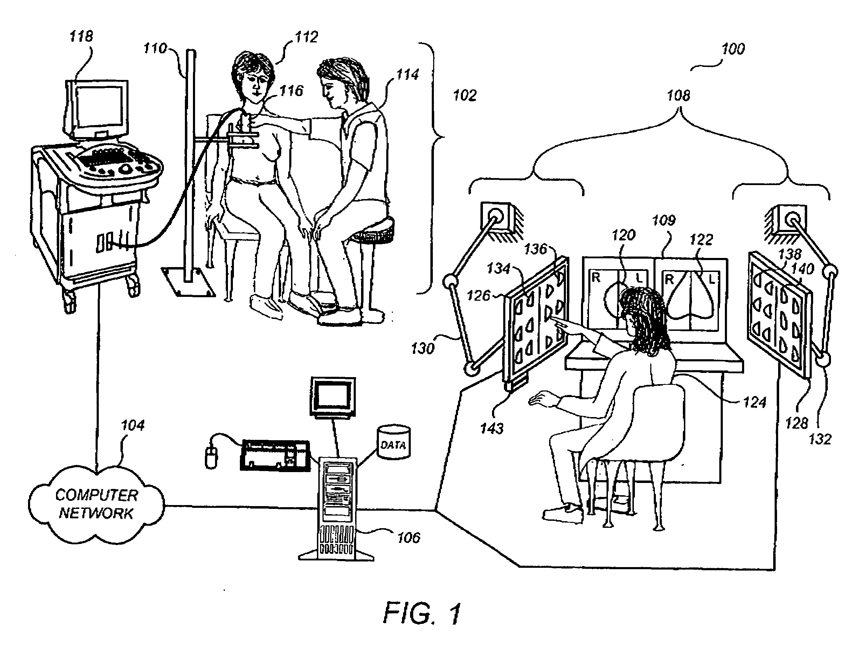

However, it is not believed that the various three-dimensional views of the "fused" data discussed in the '109 patent, such as the perspective view shown in FIG. 1 thereof, would be useful to a typical radiologist trained in conventional x-ray mammography methods. As described *supra,* radiologists typically spend many years developing expertise in analyzing a very particular set of two-dimensional x-ray mammographic data taken from standardized views, most commonly the craniocaudal (CC) and mediolateral oblique (MLO) views. It is believed that most radiologists would be reluctant to "start over again" with an entirely new, different way of viewing the complex structures of a breast, and that the medical industry would likewise be reluctant to force radiologists to accept these viewing methods.

It would be further desirable to provide an adjunctive ultrasound mammography system that displays breast ultrasound information in a manner that facilitates the radiologist's perception of internal breast structures that may not be readily apparent in an x-ray mammogram, while also being able to confirm the radiologist's perception of internal breast structures that are apparent in the x-ray mammogram.

It would be even further desirable to provide an adjunctive ultrasound mammography system that displays breast ultrasound information in a manner that supplements, rather than replaces, conventional x-ray mammogram viewing methods, thereby increasing the likelihood of adoption by both individual radiologists and the medical industry.

It would be even further desirable to provide an adjunctive ultrasound mammography system that takes little or no special familiarization or training from the radiologist in order to effectively view breast ultrasound information.

It would be still further desirable to provide an interactive user interface for an adjunctive ultrasound mammography system that allows the radiologist to quickly and intuitively navigate among different representations of the breast ultrasound information

It would be even further desirable to display such breast ultrasound information in a manner that allows benign features to be more easily dismissed by the viewing radiologist.

DE 19902521 A1 discusses an ultrasound mammography device in which a tensioned for made from ultrasound-transparent material is arranged under and essentially parallel to an upper compression plate which is displaceable relative to a ower compression plate. A liquid coupling layer is provided between the foil and the upper compression plate to directly contact an ultrasonic transducer for improved acoustic coupling.

### SUMMARY

A device according to the invention is defined in claim 1. Preferred embodiments are defined in the dependent claims. Many of the practical barriers to widespread integration of ultrasound mammography into existing mass breast cancer screening environments are mitigated. Additionally, many of the medical community acceptance barriers, economic barriers, and other political barriers to widespread integration of ultrasound mammography into existing mass breast cancer screening environments are mitigated.

The scanning apparatus of the preferred adjunctive ultrasound mammography system is configured to yield ultrasound slices from successive planes in a breast volume substantially parallel to a plane of a predetermined x-ray mammogram view of the breast. The scanning apparatus supports and maintains the breast during the ultrasound scan in a manner that promotes volumetric thoroughness of the scan, with the resulting ultrasound slices extending substantially all the way to the chest wall, The scanning apparatus is capable of partially flattening the breast according to a desired x-ray mammogram view plane while also maintaining patient comfort. Efficient patient throughput is facilitated, while at the same time the risk of inter-patient contamination and fomite propagation is minimized.

According to another preferred embodiment, the scanning apparatus comprises first and second compressive members that sandwich the breast along a plane that is near a standard x-ray mammogram view plane. The first compressive member is movable with respect to the second compressive member to allow entry of the breast therebetween, and preferably comprises a conformable sheet of acoustically transparent material in a taut state. An inner surface of the first compressive member compresses the breast while an outer surface accommodates an ultrasound probe that scans the compressed breast. Preferably, the first compressive member forms a first shallow angle with respect to the standard x-ray mammogram view plane such that interrogating ultrasonic waves from the ultrasound probe can penetrate through to the chest wall. This enhances image quality near the chest wall while still providing an overall mammogram-like view of the breast. Preferably, a nipple support element is provided on the second compressive member that urges the nipple into acoustic communication with the first compressive member. This enhances ultrasonic imaging of the breast nipple, which is a meaningful reference point in comparing the ultrasound mammography results to the x-ray mammography results. The second compressive member may comprise a substantially rigid surface and/or may comprise an air bag, a fluid bag, or a preformed sponge-like material designed to promote patient comfort while also providing a sufficient degree of breast compression:

According to a preferred embodiment, the adjunctive ultrasound display apparatus provides an array of thick-slice thumbnail images, each thick-slice thumbnail image comprising information integrated from a plurality of adjacent ultrasound slices and representing a thick-slice or slab-like portion of the breast volume substantially parallel to the standard x-ray mammogram view. The adjunctive ultrasound display apparatus comprises one or more adjunct display monitors positioned near a conventional x-ray mammogram display such that a screening radiologist can quickly turn their attention to the thick-slice thumbnail images to clarify questionable portions of the x-ray mammogram. In one preferred embodiment, each thick-slice thumbnail image is positioned not more than twenty inches from its corresponding x-ray mammogram view. Whereas the x-ray mammogram only shows the overall sum of breast tissue densities, the ultrasound thick-slice thumbnail images permit a quick view of individual thick-slice portions of the breast tissue. This allows the radiologist, for example, to quickly investigate whether a suspicious-looking mass in the x-ray mammogram is truly a tumor or is simply a coincidental confluence of patterns from different breast planes.

### BRIEF DESCRIPTION OF THE DRAWINGS

FIG. 1 illustrates a conceptual diagram of a system and method for breast cancer screening using adjunctive ultrasound mammography according to a preferred embodiment;

FIG. 2 illustrates a simplified perspective view of an ultrasound scanning apparatus according to a preferred embodiment;

FIG. 3A illustrates a side view of a breast compressed within the ultrasound scanning apparatus of FIG. 2;

FIG. 3B illustrates a simplified perspective view of a portion of an ultrasound scanning apparatus according to a preferred embodiment;

FIG. 3C illustrates a side view of a breast compressed within the ultrasound scanning apparatus of FIG. 3B; and

FIG. 3D illustrates a conceptual top view of an ultrasound scanning apparatus according to a preferred embodiment that facilitates an arcuate scanning trajectory

### DETAILED DESCRIPTION

FIG. 1 illustrates a conceptual diagram of a system **100** and associated methods for breast cancer screening using adjunctive ultrasound mammography according to a preferred embodiment. Adjunctive ultrasound mammography refers to the acquisition and display of breast ultrasound information during the breast cancer screening process in a manner that supplements x-ray mammogram information. System **100** comprises an ultrasound scanning station **102**, a computer network **104**, an adjunctive ultrasound server 106, and an adjunctive ultrasound screening station **108**. Ultrasound scanning station **102** comprises an ultrasound scanning apparatus **110** for facilitating breast ultrasound scans of the patient **112** by an ultrasound technician **114**. An ultrasound probe **116** is used to scan a breast of the patient **112**, with reflected acoustic interrogation signals being processed by an ultrasound machine **118**.

The ultrasound scanning apparatus **110** supports and maintains the breast during the ultrasound scanning process. According to a preferred embodiment, the ultrasound scanning apparatus **110** also flattens the breast along a plane parallel to a standard x-ray mammogram view plane such that resulting ultrasound images correspond more closely to standard x-ray mammogram images. In the example of FIG. 1, the standard x-ray mammogram view is the craniocaudal (CC) view. While described herein with respect to the CC view for simplicity and clarity of explanation, it is to be appreciated that the preferred embodiments are readily applied to the mediolateral oblique (MLO) view or to standard or custom x-ray mammogram views.

Although not shown in FIG. 1, the patient 112 also undergoes a standard x-ray mammography procedure in addition to the ultrasound mammography procedure. The x-ray mammogram is usually taken during the same office visit as the ultrasonic mammography scans, although the scope of the preferred embodiments is not so limited. The ultrasound technician 114 may be the same person or a different person as the x-ray technician who performs the x-ray mammography procedure.

If the ultrasound probe 116 is manipulated by hand, as in the embodiment of FIG. 1, a position sensing system (not shown) is used to track the probe position such that the acquired ultrasound frames may be processed into a three-dimensional volumetric representation of the breast. It is generally preferable, however, that the ultrasound probe 116 be machine-manipulated and controlled so as to provide reliable, consistent ultrasound scans. The ultrasound scans should be of sufficient resolution and taken at small enough intervals such that the three-dimensional volumetric representation has sufficient resolution to enable computer-aided diagnosis (CAD) algorithms to perform effectively, and such that both individual ultrasound slices and thick-slice images are of sufficient resolution to enable meaningful screening assistance to the radiologist.

As will be described further infra, the raw ultrasound scans may be taken directly in the standard x-ray mammogram view plane, or may alternatively be taken from a different orientation. When the raw ultrasound scans are taken directly in the standard x-ray mammogram view plane, each individual ultrasound slice is computed directly from an acquired two-dimensional ultrasound image or ultrasound frame. When the raw ultrasound scans are taken from a different orientation, each individual ultrasound slice corresponds to a plane of voxels (volume elements) in a three-dimensional volumetric representation of the breast, the plane of voxels being oriented in a direction parallel to the standard x-ray mammogram view plane. Most commonly, the three-dimensional volumetric representation of the breast is computed from the raw ultrasound scans, and then the individual ultrasound slice is extracted therefrom. However, in other preferred embodiments, it is not always necessary to reconstruct the entire three-dimensional volumetric representation to compute the individual ultrasound slices. Stated more generally, if the raw ultrasound scans are taken in planes directly parallel to a plane of interest (CC, MLO, or a different "custom" plane of importance), each individual ultrasound slice is computed directly from an acquired two-dimensional ultrasound image or ultrasound frame, whereas if the raw ultrasound scans are taken from directions different than the plane of interest, each individual ultrasound slice corresponds to a plane of voxels in a three-dimensional volumetric representation of the breast in a direction parallel to the plane of interest

Ultrasound machine 118 may generally comprise any commercially available ultrasound machine having sufficient resolution, speed, and network connectivity to achieve the functionalities described herein. In one preferred embodiment, ultrasound machine 118 comprises a system available from U-Systems, Inc. of San Jose, California. Preferably, ultrasound probe 116 is of sufficient width (e.g., 15 cm) to scan the entire flattened breast surface in a single sweep. If a smaller probe (e.g., 7.5 cm) is used for cost-containment reasons or other practical reasons, the ultrasound probe 116 is swept across two or more times until the entire breast surface is scanned.

During or after the ultrasound scanning process, the raw ultrasound data is provided across the computer network 104 to the adjunctive ultrasound server 105, where the raw ultrasound data is processed into adjunctive ultrasound data that will be made available to the screening radiologist, the adjunctive ultrasound data including ultrasound slices, thick-slice images, vibrational Doppler imaging (VDI) images, CAD outputs, and other useful information. It is to be appreciated that the processing of the raw ultrasound data into the adjunctive ultrasound data may be performed by any of a variety of different computing devices coupled to the computer network 104 and then transferred to the adjunctive ultrasound server 106.

In current mass breast cancer screening environments based on x-ray mammography, a screening radiologist 124 examines x-ray mammograms for many patients en masse in a single session using an x-ray viewing station 109. The x-ray viewing station 109 may range from a simple light box, as in FIG. 1, to more complex x-ray CAD workstations that automatically move the x-ray mammograms past the radiologist 124 on a conveyor belt as a nearby CAD display highlights suspicious areas of the mammogram. Almost universally, left and right CC x-ray views 120 are positioned on one side of the x-ray viewing station 109, and left and right MLO x-ray views 122 are positioned on the other side. The radiologist 124 quickly examines the x-ray mammograms. For some x-ray mammograms the radiologist needs only a few seconds, while for other x-ray mammograms the radiologist needs up to five minutes, with an average being about two minutes per mammogram.

According to a preferred embodiment, this existing arrangements remains substantially undisturbed, but is augmented with equipment and data that facilitates fast and thorough x-ray mammogram screening by giving the radiologist a quick ultrasonic "second look" at the internal breast structure. Adjunctive ultrasound screening station 108 comprises an adjunct display 126 conveniently positioned near the x-ray viewing station 109 such that the radiologist 124 can easily look back and forth between the adjunct display 126 and the x-ray mammograms 120 and 122. Thumbnail representations 134 and 136 of thick-slice images are displayed. Generally speaking, a thick-slice image is an integration of a plurality of substantially parallel individual ultrasound slices used to represent a slab-like or thick-slice volume of the breast. The thickness of the slab-like or thick-slice volume may lie, for example, in the range of 2 mm to 20 mm, although the scope of the preferred embodiments is not so limited. Techniques for integrating the component ultrasound slices into thick-slice images according to the preferred embodiments include arithmetic averaging, geometric averaging, reciprocal averaging, exponential averaging, and other averaging methods, in each case including both weighted and unweighted averaging techniques. Other suitable integration methods may be based on statistical properties of the population of component ultrasound slices at common locations, such as maximum value, minimum value, mean, variance, or other statistical algorithms.

Preferably, the thick-slice images correspond to slab-like or thick-slice volumes of the breast having a thickness related to the size of the lesions to be detected. At an upper end, a larger thickness of 20 mm, for example, may be used if it is desirable to overlook most of the breast details and direct the user's attention to larger features on the order 10 mm in size. At a lower end, a smaller thickness of 3 mm, for example, may be used if it is desirable to view small, low-contrast lesions on the order of 2 mm in size. Thicknesses in the range of 7 mm - 12 mm are likely to be suitable for most breast cancer screening purposes.

For even quicker reference, a second adjunct display 128 is provided to form an adjunct display pair, each side corresponding to the nearest mammogram view being displayed at x-ray viewing station 109. A bar code reader 143 reads a bar code of the x-ray mammogram, wherein the associated adjunctive ultrasound data for that breast is automatically retrieved from the ultrasound server 106 and displayed on the adjunct displays 126 and 128.

By way of example and not by way of limitation, where the x-ray mammogram 120 represents the craniocaudal (CC) x-ray view for right and left breasts, respectively, the thumbnail thick-slice images 134 and 136 represent thick-slice portions of the right and left breast volumes, respectively, oriented parallel to the CC view plane. Where the x-ray mammogram 122 represents the mediolateral oblique (MLO) x-ray view for right and left breasts, respectively, thumbnail thick-slice images 138 and 140 displayed on adjunct display 128 represent thick-slice portions of the right and left breast volumes, respectively, oriented parallel to the MLO view plane.

Each thick-slice image usually represents between 0.5 cm to 1.0 cm of breast thickness. It has been found that this thickness range yields good results in assisting in the recognition of tumors that are of concern in the breast cancer screening process, which are about 0.5 cm or greater in diameter with an average diameter of about 1.2 cm. Also, because the flattened breast is usually somewhere between 4 cm and 6 cm thick, it has been found that the preferred 0.5 cm-1.0 cm thickness dimension facilitates an easy "single-glance" view of the entire breast, with a simple display of six to eight thick-slice images covering the entire breast volume. This is clearly advantageous over ultrasonic screening methods that examine one ultrasound slice at a time, in which case it would take up to 500 ultrasound slices or more to cover the entire breast volume. Although a 0.5-1.0 cm slab thickness and a 6-8 image layout has been found to yield good results, it is to be appreciated that the scope of the preferred embodiments is not so limited, and that a wide range of slab thicknesses and images-per-layout are within the scope of the preferred embodiments.

Each adjunct display 126 and 128 is flexibly mounted using adjustable mountings 130 and 132 such that the radiologist 124 may freely move them into different positions near the x-ray mammogram views to facilitate optimal back-and-forth viewing. It has been found that ideal back and forth viewing is facilitated where each thick-slice image is not greater than 20 inches from its corresponding x-ray mammogram image.

Preferably, the adjunct displays 126 and 128 comprise touchscreen monitors. When the radiologist 124 presses one of the thumbnail thick-slice images, a larger thick-slice image is displayed. In one preferred embodiment, the larger thick-slice image has a size identical to that of the x-ray mammogram image for further facilitating back-and-forth image comparisons. A control panel (not shown in FIG. 1 but described further infra) is positioned near or integrated with each adjunct display 126 and 128 that provides a user interface for the radiologist 124. In conjunction with the touchscreen display, the control panel permits quick manipulation and examination of the thick-slice images in a manner that facilitates rapid screening. By way of example, the radiologist 124 is permitted to examine each individual component slice of any thick-slice image, jogging through the individual ultrasound slices or viewing a cine-loop representation. Also, as will be described further infra, the radiologist may flexibly overlay useful information onto the thick-slice images such as vibrational Doppler image (VDI) outputs and computer-aided diagnosis (CAD) outputs.

Thus, an adjunctive ultrasound system according to the preferred embodiments does not supplant existing x-ray mammogram screening methods. Indeed, reference to the adjunctive ultrasound data is optional depending on the contents of the x-ray mammogram image, and for many patients it may not even be used at all. Rather, the adjunctive ultrasound system is there to assist the radiologist in performing their pre-existing professional duties with respect to "difficult" or "marginal" mammograms. As such, a medical establishment faces little risk of failure in acquiring an adjunctive ultrasound system according to the preferred embodiments. In a worst-case scenario, the adjunctive ultrasound system would be met with indifference by the entrenched "x-ray-only" believers, because it would not disturb their pre-existing routines. However, the adjunctive ultrasound system will be there standing by to assist in the "difficult" cases, and it is expected that even the "x-ray-only" believers will eventually find the system useful and will increasingly rely on it to increase their sensitivity and specificity performance.

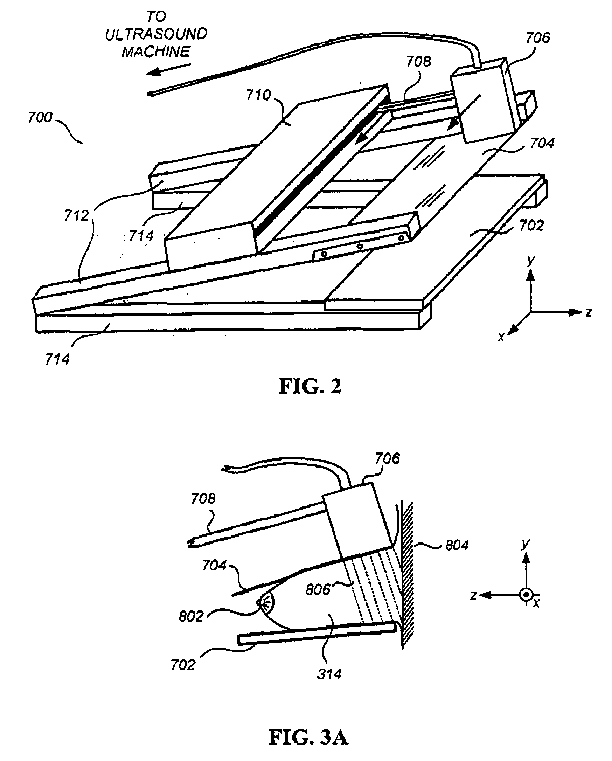

FIG. 2 illustrates a perspective view of an ultrasound scanning apparatus 700 according to a preferred embodiment of the invention Scanning apparatus 700 comprises an upper support frame 712 that is movable toward a lower support frame 714. It is not required that the upper and lower support frames be hingeably connected to each other, as implied in the configuration of FIG.2 provided that they can ultimately be brought together to so as to compress the breast. A lower compressive member 702, comprising for example a stiff Buna-N rubber material, is supported by the lower support frame 714. An upper compressive member 704 comprising a taut, partially conformable sheet of acoustically transparent material such as Mylar® polyester film is stretched across, and supported by, the upper support frame 712. An ultrasound probe 706 coupled to an ultrasound machine is positioned over the polyester film 704 and is affixed to a rigid member 708, which is in turn coupled to an actuator 710 as shown. Preferably, the polyester film 704 is optically transparent so that the medical technical can see the breast from above as it is being compressed. Preferably, as is detailed further with respect to FIG. 3A below, ultrasound scanning apparatus 700 is configured and dimensioned such that lower compressive member 702 is at a shallow angle of about 10 degrees with respect to the horizontal. Also, the polyester turn 704 preferably forms about a 5 degree angle with respect to the lower compressive member 702 when lowered to a distance of about 6 cm therefrom. While being shown between two lateral frame members in the embodiment of FIG.2 the polyester film 704 can be framed on all sides in an alternative preferred embodiment.

The lower compressive member 702 is preferably maintained at a height that allows a standing patient to lay their breast thereon without crouching and such that their chest wall is substantially vertical. In operation, the polyester film 704 is generously coated on both sides with an ultrasonic gel, mineral oil, and/or water and lowered onto the breast. Although the polyester film 704 deforms by a small amount, a substantially horizontal surface is still provided for mechanical translation of the ultrasound probe 706 across the top surface thereof. Preferably, an overall compressive force similar to that applied during x-ray mammography, e.g. about 20-25 pounds, is applied by the polyester film 704. Between patients, the polyester film 704 is replaced and the lower compressive member 702 is sterilized or replaced to prevent inter-patient contamination and fomite propagation. As is common in the field, a small metallic object such as a small ball bearing or BB is taped to the breast at a location corresponding to the nipple so that the nipple location is detectable in the acquired ultrasound images.

According to another preferred embodiment, the ultrasound probe 706 actually comprises two mechanically affixed side-by-side ultrasound probe heads, including a lower-frequency probe head and a higher-frequency probe head. Each probe head is connected to a different probe input of the ultrasound machine, which is capable of being controlled by the ultrasound technician so as to selected one probe head or the other. According to a preferred embodiment, the lower-frequency probe is used for patients with large breasts, while the higher-frequency probe is used for patients with medium or small breasts. The lower-frequency probe operates at lower frequencies, e.g. 5-7 MHz, for enabling deeper scans for the larger breast, e.g. as deep as 10 cm, albeit at a somewhat reduced resolution. The higher-frequency probe operates at higher frequencies, e.g. 8-12 MHz, which penetrates only to the shallower depths of 4-6 cm, but which provides higher resolution than the lower-frequency probe. In an alternative preferred embodiment, large breasts are scanned in a two-sweep process, the first sweep being high-frequency and the second sweep being low-frequency, and the near field portions of the images resulting from the first sweep are stitched to the far field portions of the images resulting from the second sweep.

In still another alternative embodiment, the lower compressive member 702 is replaced by a lower film/probe/actuator assembly ("lower assembly") that mirrors the upper film/probe/actuator assembly 704/706/708/710 ("upper assembly"). The lower assembly is similar to the upper assembly, and both are equipped with higher-frequency ultrasound probes. When small or medium breasts are presented, only the upper assembly actively scans the breast, and the lower assembly remains dormant, that is, it serves the function of a lower compressive member but does not actively scan the breast. However, when a large breast is presented, a first sweep of the upper assembly and a second sweep of the lower assembly are executed. The near-field portions of the images resulting from the first and second sweeps, which collectively scan the entire large breast volume, are then stitched together.

According to another preferred embodiment, the ultrasound probe 706 actually comprises two mechanically affixed end-to-end ultrasound probe heads, each having a shorter length individually (e.g., 7.5 cm) but, when placed end-to-end, forming a larger effective probe head (e.g., 15 cm). In one preferred embodiment, the first probe head is operative on a first sweep across the breast, while the second probe is operative on a second sweep. An A-B switch is provided in the ultrasound machine for automatically switching between the probe heads. In another preferred embodiment in which only a single sweep is required, the probe heads operate sequentially at non-overlapping time intervals for each ultrasound slice during the sweep. The resulting separate ultrasound frames are stitched together using known methods. Generally speaking, acquiring two shorter end-to-end probe heads is less expensive than acquiring a single long probe head. This can be appreciated with respect to transducer element yields during probe manufacture. Other factors being equal, if a single 7.5 cm transducer can be made with a 90% element yield, a 15 cm transducer would only have a 90% x 90% or 81 % element yield, with associated higher manufacturing costs.

In still another preferred embodiment, an irrigation mechanism (not shown) is provided that maintains a stream of nonviscous, acoustically transparent fluid such as water at an interface between the thin film 704 and the ultrasound probe 706 during the ultrasound scans, thereby enhancing the acoustic coupling between said ultrasound probe 706 and the breast. In still another preferred embodiment, one or more audio frequency transducers (not shown) are affixed to one or more of the lower compressive member 702 and the thin film 704 for inducing audio frequency vibrations in the breast during the ultrasound scans according to a vibrational Doppler imaging (VDI) modality.

In yet another preferred embodiment, the upper support frame 712 is springably and hingeably mounted with respect to the lower support frame 714 such that the polyester film 704 may adaptably tilt forward or backward in the anterior/posterior direction relative to the patient as it compresses the breast to obtain a best compression angle. Visualized with respect to FIG. 2, the springable, hingeable tilting would take place around an axis parallel to the "x" axis, and that axis of rotation would itself be movable in the "y" direction and, to a lesser extent, in the "z" direction. Such a device would provide enhanced compression for a wider variety of breasts that have differing profiles in the y-z plane of FIG. 2 In one preferred embodiment, the device is similar to a commercially available conforming compression device used for x-ray mammography, termed the FAST Paddle (Fully Automatic Self-adjusting Tilt Compression Paddle), available from LORAD, a Hologic Company, of Danbury, Connecticut, and described on their public web site.

In still another preferred embodiment, a gap-filling gel bag or other flexible bag containing an acoustically transparent fluid such as water, gel, mineral oil, or the like is positioned above the breast prior to compression by the polyester film 704. This enhances imaging of those breast portions corresponding to upper skin locations that may not come directly into contact with the polyester film 704 during compression. The gap-filling bag surface may comprise latex, Nitrile, Saran Wrap, cellophane, or other suitable material. Alternatively, the gap filling material may be a highly conformable cross-linked polymer gel (or a "slime" material often used in children's toys) having appreciable acoustic transmissivity but requiring an outer bag.

In yet another preferred embodiment, the polyester film 704 is replaced by a stiffer plastic material such as PETG. The stiffer plastic material does not require taut placement across frame members, but rather is vacuum-formed into a structure having a flat bottom surrounded by elevated outer ridges. The elevated outer ridges are affixed to the frame members. When the structure is pressed down onto the breast, a lower surface of the flat bottom compresses the breast, and the ultrasound probe is swept across an upper surface of the flat bottom. Substantial force is applied to the breast by virtue of the stiffness of the flat bottom material as strengthened by the elevated outer ridges, and the flat bottom only deforms by a small amount.

FIG. 3A illustrates a side cutaway view of a breast 314 as it is being compressed and scanned by the ultrasonic scanning apparatus 700. One advantage of the ultrasound scanning apparatus 700 is illustrated in FIG. 3A which depicts a conceptual view of the substantially vertical chest wall 804 of the patient. Because the angle of the polyester film 704 is less than 90 degrees (preferably about 75 degrees) with respect to the chest wall 804, the acoustic interrogation field 806 can penetrate all the way to the chest wall 804 for most of the breast volume. This penetration can be achieved even where a high frequency ultrasound probe (e.g., 7.5 MHz or greater), which has a greater resolution but a lesser acoustic penetration depth, is used. This feature is especially important in light of the fact that a substantial percentage of breast cancers are formed within 2 cm of the chest wall. Yet another advantage of the ultrasound scanning apparatus 700 is its physical profile as a "stand-up" apparatus that takes up minimal floor space, which is a valuable and scarce resource at many medical clinics.

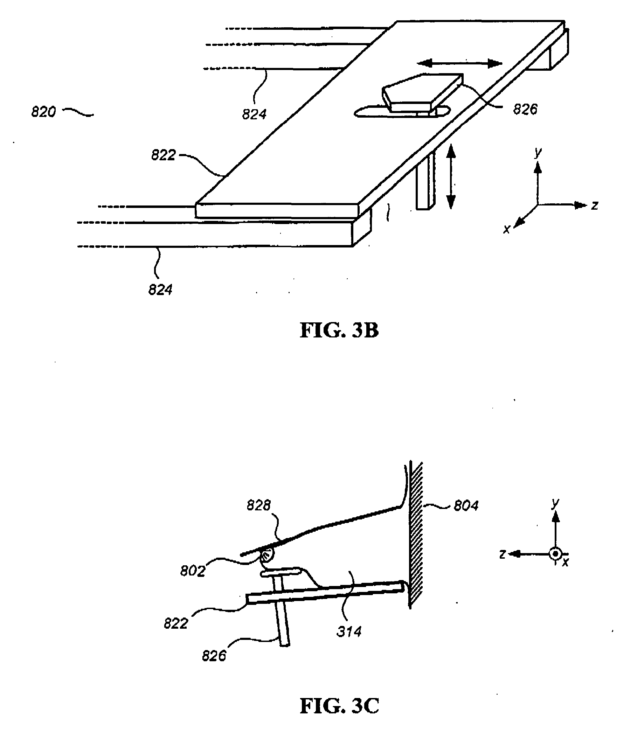

FIG. 3B illustrates a lower portion 820 of an ultrasound scanning apparatus according to another preferred embodiment in which nipple imaging is enhanced. The ultrasound scanning apparatus is similar to that of FIG.2 but only an area around the lower compressive member is drawn for clarity of description. A lower compressive member 822 is positioned on a lower support member 824 as in the embodiment of FIG. 2 Slidably coupled to the lower compressive member is a nipple support platform 826 for urging the breast nipple upward toward the polyester film. The nipple support platform 826 comprises a rigid frame that is covered by a soft, pliable silicone rubber sheet. The nipple support platform 826 is adjustable in two directions as indicated in FIG.3B in order to be operative on a variety of different breast types. A locking mechanism (not shown) locks the nipple support platform 826 into place after being adjusted to a particular breast. The nipple support platform 826 allows the area of the breast near the nipple, as well as the nipple itself, to appear more completely in the acquired adjunctive ultrasound data.

FIG. 3C illustrates a side view of the breast 314 as it is being compressed and scanned by the ultrasonic scanning apparatus of FIG. 3B. The nipple support platform 826 gently urges the nipple 802 against the polyester film 828 such that it makes sound acoustic contact therewith. This enhances imaging of the nipple 802, which is a useful reference point for the radiologist in comparing the ultrasonic images to the x-ray mammogram images. By acoustic contact, it is meant that the nipple 802 either directly touches the polyester film 828 or is close enough to it such that any gaps between the nipple 802 and the polyester film 828 are occupied by ultrasonic gel or other acoustically conductive material.

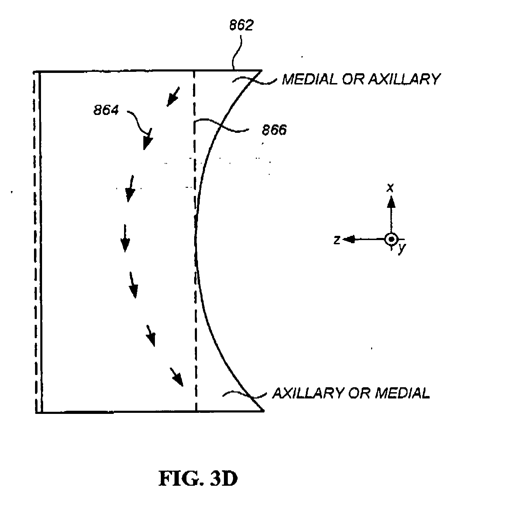

FIG. 3D illustrates a simplified top view of a lower portion of an ultrasound scanning apparatus according to another preferred embodiment in which a more thorough scan of the breast is provided toward the medial and axillary regions of the breast near the chest wall. A lower compressive member 862 is curved so as to be conformal with the chest wall all the way from the center-facing side of the breast to the axillary side of the breast. It has been found that a radius of curvature of about 9 inches provides improved chest wall and axillary imaging of the breast for a broad range of breast sizes. Optionally, different lower compressive members with different shapes and/or radii of curvature are used for different patients, e.g., different lower compressive members for small, medium, and large breasts. During the scanning process, the ultrasound probe is moved in a curved or arcuate trajectory by an actuator mechanism, for example, by adapting the actuator 710 of FIG. 2 to have an inward/outward motion along with its side-to-side motion. The increased-area region 864 is shown in FIG. 3D for comparison with a rectangular-area scan area 866. Preferably, the associated x-ray mammograms are taken using a similarly-conformal x-ray detector mechanism, such as that described in U.S. Pat. 6,181,769, which is incorporated by reference herein. This allows meaningful comparison of the increased-area adjunctive ultrasound data with corresponding increased-area x-ray mammogram data.

Whereas many alterations and modifications of the present invention will no doubt become apparent to a person of ordinary skill in the art after having read the foregoing description, it is to be understood that the particular embodiments shown and described by way of illustration are in no way intended to be considered limiting. By way of example, although described *supra* in terms of adjunctive ultrasound screening, in view of the present disclosure one skilled in the art would readily be able to apply the thick-slice display apparatus of the preferred embodiments in the context of computerized tomography (CT) and/or magnetic resonance imaging (MRI) environments. By way of further example, the preferred embodiments described *supra* may also be used with different ultrasound modalities other than B-mode scans, including power or color Doppler modalities, and may also be used in conjunction with vibrational Doppler imaging (VDI) modalities. Therefore, reference to the details of the preferred embodiments are not intended to limit their scope, which is limited only by the scope of the claims set forth below.

## Claims

1. An apparatus (700) for facilitating acquisition of ultrasound scans of a breast of a patient by an ultrasound probe (706), the apparatus (700) comprising:
a first compressive member (702) supported by a first support frame (714);
a second compressive member (704) supported by a second support frame (712) movably adjacent to said first compressive member (702) to allow placement and compression of the breast between said first and second compressive members (702,704); and
a probe translation mechanism (708,710) configured to hold the ultrasound probe (706),
**characterised in that** the second compressive member comprises a conformable, acoustically transparent membrane (704) disposed across and supported by the second support frame (712) in a substantially taut state, said membrane (704) having a first surface for contacting the breast and a second surface opposite said first surface,wherein the probe translation mechanism (710,708) is configured to hold the ultrasound probe (706) against said second surface of said membrane (704) while translating the ultrasound probe (706) thereacross, the ultrasound probe (706) being in acoustic communication with the breast through said membrane (704) while being translated.

2. The apparatus (700) of claim 1, wherein said membrane (704) is a polyester film, and wherein said first compressive member (702) comprises a substantially rigid material.

3. The apparatus (700) of claim 1 or 2, said apparatus (700) having (i) an open configuration in which said membrane (704) is sufficiently distant from said first compressive member (702) such that the breast may be placed therebetween, and (ii) a compressed configuration in which the breast is compressed between the first compressive member (702) and said membrane (704), the ultrasound probe (706) scanning the breast when said apparatus (700) is in said compressed configuration.

4. The apparatus of any preceding claim, wherein the second compressive member is configured such that the membrane (704) is replaceable between ultrasound scans.

5. The apparatus of any preceding claim, wherein said membrane (704) is optically transparent so that a medical technician can see the breast from above as it is being compressed.

## Patentansprüche

1. Vorrichtung (700) zur Erleichterung der Aufnahme von Ultraschalluntersuchungen einer Brust einer Patientin durch einen Ultraschallprüfkopf (706), wobei die Vorrichtung (700) Folgendes umfasst:
ein erstes Kompressionselement (702), das von einem ersten Tragrahmen (714) getragen wird;
ein zweites Kompressionselement (704), das von einem zweiten Tragrahmen (712) getragen wird, der beweglich neben dem ersten Kompressionselement (702) liegt, um eine Anordnung und Kompression der Brust zwischen den ersten und zweiten Kompressionselementen (702, 704) zu erlauben; und
einen Prüfkopf-Verschiebungsmechanismus (708, 710), der konfiguriert ist, um den Ultraschallprüfkopf (706) zu halten,
**dadurch gekennzeichnet, dass** das zweite Kompressionselement eine anpassbare, akustisch transparente Membran (704) umfasst, die über dem zweiten Tragrahmen (712) angeordnet ist und von diesem in einem im Wesentlichen gespannten Zustand getragen wird, wobei die Membran (704) eine erste Fläche zum Kontakt mit der Brust und eine zweite Fläche, die der ersten Fläche gegenüberliegt, aufweist, wobei der Prüfkopf-Verschiebungsmechanismus (710, 708) konfiguriert ist, um den Ultraschallprüfkopf (706) gegen die zweite Fläche der Membran (704) zu halten, während der Ultraschallprüfkopf (706) darüber verschoben wird, wobei sich der Ultraschallprüfkopf (706) durch die Membran (704) hindurch in akustischer Verbindung mit der Brust befindet, während er verschoben wird.

2. Vorrichtung (700) nach Anspruch 1, wobei die Membran (704) eine Polyesterfolie ist, und wobei das erste Kompressionselement (702) ein im Wesentlichen unbiegsames Material umfasst.

3. Vorrichtung (700) nach Anspruch 1 oder 2, wobei die Vorrichtung (700) (i) eine offene Konfiguration aufweist, in der die Membran (704) weit genug von dem ersten Kompressionselement (702) entfernt ist, so dass man die Brust dazwischen anordnen kann, und (ii) eine komprimierte Konfiguration aufweist, in der die Brust zwischen dem ersten Kompressionselement (702) und der Membran (704) komprimiert ist, wobei der Ultraschallprüfkopf (706) die Brust untersucht, wenn die Vorrichtung (700) sich in der komprimierten Konfiguration befindet.

4. Vorrichtung nach einem der vorhergehenden Ansprüche, wobei das zweite Kompressionselement derart konfiguriert ist, dass die Membran (704) zwischen den Ultraschallabtastungen austauschbar ist.

5. Vorrichtung nach einem der vorhergehenden Ansprüche, wobei die Membran (704) optisch transparent ist, so dass ein medizinisch-technischer Assistent die Brust von oben sehen kann, während sie komprimiert wird.

## Revendications

1. Dispositif (700) pour faciliter l'acquisition d'images à ultrasons du sein d'une patiente par une sonde à ultrasons (706), le dispositif (700) comportant :
un premier élément de compression (702) supporté par une première structure de support (714) ;
un second élément de compression (704) supporté par une seconde structure de support (712) adjacente de façon mobile au dit premier élément de compression (702) afin de permettre le placement et la compression du sein entre lesdits premier et second éléments de compression (702, 704) ; et
un mécanisme de translation de sonde (708, 710) configuré pour soutenir la sonde à ultrasons (706), **caractérisé en ce que** le second élément de compression comprend une membrane de forme adaptable, acoustiquement transparente (704) disposée à travers, et supportée par, la seconde structure de support (712) dans un état essentiellement tendu, ladite membrane (704) présentant une première surface permettant de se trouver en contact avec le sein et une seconde surface opposée à ladite première surface, dans lequel le mécanisme de translation de la sonde (710, 708) est configuré pour maintenir la sonde à ultrasons (706) contre ladite seconde surface de ladite membrane (704) tout en translatant la sonde à ultrasons (706) d'un côté à l'autre, la sonde à ultrasons (706) étant en communication acoustique avec le sein à travers ladite membrane (704) tout en étant translatée.

2. Dispositif (700) selon la revendication 1 dans lequel ladite membrane (704) est un film de polyester et dans lequel ledit premier élément de compression (702) comprend un matériau essentiellement rigide.

3. Dispositif (700) selon la revendication 1 ou 2, ledit dispositif (700) présentant (i) une configuration ouverte dans laquelle ladite membrane (704) se trouve à une distance suffisante dudit premier élément de compression (702) de telle façon que le sein puisse être placé entre eux, et (ii) une configuration de compression dans laquelle le sein est comprimé entre le premier élément de compression (702) et ladite membrane (704), la sonde à ultrasons (706) analysant le sein lorsque ledit dispositif (700) se trouve dans ladite configuration de compression.

4. Dispositif selon l'une quelconque des revendications précédentes, dans lequel le second élément de compression est configuré de façon que la membrane (704) puisse être remplacée entre les analyses par ultrasons.

5. Dispositif selon l'une quelconque des revendications précédentes, dans lequel ladite membrane (704) est optiquement transparente de sorte qu'un technicien médical puisse voir le sein à partir de dessus lorsqu'il est comprimé.
